(19) Europäisches Patentamt
European Patent Office
Office européen des brevets

(11) Veröffentlichungsnummer: **0 684 231 A1**

(12) # EUROPÄISCHE PATENTANMELDUNG

(21) Anmeldenummer: **95107887.2**

(22) Anmeldetag: **24.05.95**

(51) Int. Cl.[6]: **C07D 209/34**, C07D 215/50, A61K 31/40, A61K 31/47

(30) Priorität: **27.05.94 CH 1649/94**

(43) Veröffentlichungstag der Anmeldung: **29.11.95 Patentblatt 95/48**

(84) Benannte Vertragsstaaten: **AT BE CH DE DK ES FR GB IT LI NL SE**

(71) Anmelder: **Cerbios-Pharma S.A.**
**Via Pian Scairolo 6**
**CH-6917 Barbengo (CH)**

(72) Erfinder: **Burger, Ulrich, Prof.**
**3, Chemin de la Foret**
**CH-1222 VNsenaz**
**GenCve (CH)**
Erfinder: **Jaton, Jean-Claude, Prof.**
**Avenue Adrien-Jeandin 14**
**CH-1226 Thonex (CH)**
Erfinder: **Marazza, Fabrizio, Dr.**
**Rombosco**
**CH-6986 Novaggio (CH)**
Erfinder: **Lewenstein, Ari, Dr.**
**Freigutstrasse 10**
**CH-8002 Z rich (CH)**
Erfinder: **Sirotnak, Francis M.**
**Throop**
**Pennsylvania (US)**

(74) Vertreter: **Blum, Rudolf Emil Ernst**
**c/o E. Blum & Co**
**Patentanwälte**
**Vorderberg 11**
**CH-8044 Zürich (CH)**

(54) **Pharmazeutisches Präparat zur Modulierung der Immunreaktion und neue Indol- und Chinolinderivate.**

(57) Es werden pharmazeutische Präparate zur Modulierung der Immunreaktion warmblütiger Tiere beschrieben, die als Wirkstoff Derivate der 2-Oxindol-3-essigsäure oder der 2-Oxo-1,2,3,4-tetrahydrochinolin-4-carbonsäure enthalten, und wobei die Derivate gegebenenfalls miteinander im Gleichgewicht stehen können.

Die in den pharmazeutischen Präparaten enthaltenen Derivate umfassen solche Verbindungen, die bereits in der Literatur beschrieben sind und auch neue Verbindungen.

Des weiteren werden neue Derivate der 2-Oxindol-3-essigsäure und der 2-Oxo-1,2,3,4-tetrahydrochinolin-4-carbonsäure beschrieben, insbesondere auch die aus dem Enantiomerengemisch isolierten reinen Antipoden der unsubstituierten 1-Oxo-1,2,3,4-tetrahydrochinolin-4-carbons äure.

EP 0 684 231 A1

Die vorliegende Erfindung betrifft pharmazeutische Präparate zur Modulierung der Immunreaktion warmblütiger Tiere, die als Wirkstoffe Derivate der 2-Oxindol-3-essigsäure oder analoge Derivate der 2-Oxo-1,2,3,4-tetrahydrochinolin-carbonsäure enthalten, die im Gleichgewicht miteinander stehen können und wobei an diese Derivate allenfalls auch ein oder mehrere Reste einer Hexose gebunden sein können. Die in den pharmazeutischen Präaraten enthaltenen Wirkstoffe umfassen sowohl neue Wirktoffe als auch solche, die bereits in der Literatur beschrieben sind, jedoch bisher noch nie als Wirkstoffe irgendwelcher pharmazeutischer Präparate eingesetzt wurden.

Des weiteren betrifft die vorliegende Erfindung neue Derivate der 2-Oxindol-3-essigsäure, bzw. der 2-Oxo-1,2,3,4-tetrahydrochinolin-4-carbonsäure.

## BESCHREIBUNG DES STANDES DER TECHNIK

Pharmazeutische Präparate, die als Wirkstoff Verbindungen enthalten, die sich von der 1-Oxindol-3-essigsäure, bzw. der 2-Oxo-1,2,3,4-tetrahydrochinolin-4-carbonsäure ableiten, sind bisher nicht beschrieben worden. Solche Verbindungen, welche sich in der Grundstruktur von den hier genannten 2-Oxo-1,2,3,4-tetrahydrochinolin-carbonbonsäure dadurch unterscheiden, dass sie in dem heterocyclischen Rest eine Doppelbindung aufweisen und somit Derivate der 2-Oxo-1,2-dihydrochinolin-4-carbonsäure darstellen und die ferner nicht als freie Säuren, sondern in Form von Estern mit einer Alkoholkomponente vorliegen, in welcher die Hydroxygruppe an einen basischen Azabicycloalkylrest gebunden ist, wurde von Hayashi, Hiroaki et al. auf ihre Aktivität als 5'-HT3 Rezeptor-antagonisten getestet. Es sei in diesem Zusammenhang auf Chemical Abstract, Vol. 118, Nr. 5, 1. Februar 1993, Columbus, Ohio, US, Abstract Nr. 38750n verwiesen, welches ein Abstract der Originalarbeit in J. Med. Chem. 1992, 35(26), 4893-902 darstellt.

Eine Ahzahl der in den erfindungsgemässen pharmazeutischen Präparaten als Wirkstoffe eingesetzten Derivate der 2-Oxindol-3-essigsäure, bzw. der analogen Derivate der 2-Oxo-1,2,3,4-tetrahydrochinolin-4-carbonsäure sind jedoch keine neuen chemischen Verbindungen, sondern sie wurden bereits in der Literatur beschrieben.

Die unsubstituierte 2-Oxindol-3-essigsäure und die unsubstituierte 2-Oxo-1,2,3,4-tetrahydrochinolin-4-carbonsäure sind bereits in der Literatur beschrieben, und diese Verbindungen weisen die in der Folge angegebenen Formeln Ia und IIa auf.

Es ist ausserdem seit langem bekannt (siehe die Veröffentlichung von A.J. Hill et al., Am. Chem. J., vol. 52, Seiten 769-775, 1930), dass unter hydrolytischen Bedingungen die 2-Oxindol-3-essigsäure der Formel Ia im Gleichgewicht mit der 2-Oxo-1,2,3,4-tetrahydrochinolin-4-carbonsäure der Formel IIa steht, und diese Gleichgewichtsreaktion wird durch eine Oeffnung des Indolringes und einen ringerweiternden Ringschluss nach dem folgenden Reaktionsschema erläutert:

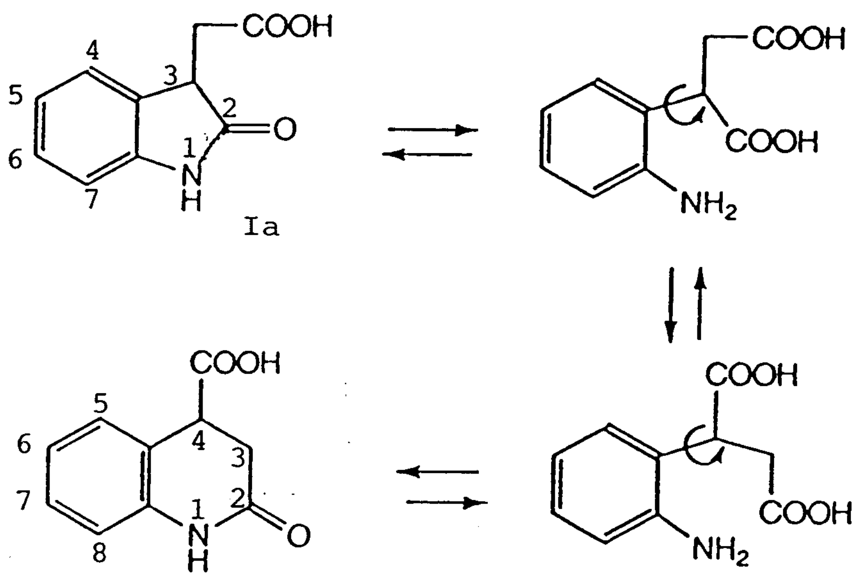

Ia

IIa

Unter sauren Bedingungen ist das Gleichgewicht stark auf die Seite von IIa verschoben.

Es ist ferner bekannt, dass die Indol-3-essigsäure, welche sich von der Verbindung der oben angegebenen Formel Ia dadurch unterscheidet, dass in der 2-Stellung des Indolkernes eine $CH_2$-Gruppe statt der Carbonylgruppe vorliegt, ein Pflanzenwachstumsfaktor ist, der von Maiskeimlingen über die 2-Oxindol-3-essigsäure der oben angegebenen Formel Ia schliesslich in das entsprechende Derivat mit einem Hydroxysubstituenten in der 7-Stellung des Indolgerüstes und anschliessend in das entsprechende Glykosid umgewandelt wird (siehe die Veröffentlichung von H. M. Nonhebel et al. in The Journal of Biological Chemistry, Vol. 260, Seiten 12685-12689, 1985 und das entsprechende Abstract in Chemical Abstracts, Vol.103, Nr. 23, 9. Dezember 1985, Columbus, Ohio, US; Abstract Nr. 193222a.

Die folgenden Derivate der 2-Oxindol-3-essigsäure der Formel I, bzw. der 2-Oxo-1,2,3,4-tetrahydrochinolin-4-carbonsäure der Formel II,

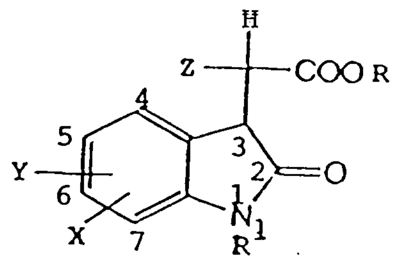

I

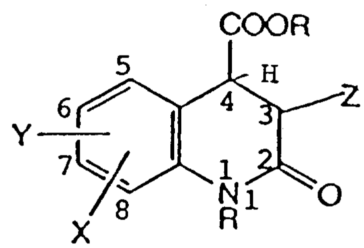

II

sind bereits in der Literatur beschrieben:

Von den freien Carbonsäuren, in welchen also R ein Wasserstoffatom ist, diejenigen Verbindungen, in welchen Z ein Wasserstoffatom darstellt und $R^1$ ebenfalls ein Wasserstoffatom ist, wobei die Reste X und Y beide Wasserstoff bedeuten, oder die Reste X und Y Chloratome oder Bromatome in den Stellungen 6 und 8 des Benzolkernes des Tetrahydrochinolinderivates der Formel II bedeuten oder einer der Reste X und Y ein Bromatom oder ein Jodatom in der Stellung 6 des Tetrahydrochinolingerustes und der andere ein Wasserstoffatom bedeutet, und

diejenigen Derivate der 2-Oxindol-3-essigsäure der Formel I, in welcher R, $R^1$ und Z Wasserstoff bedeuten und

einer der Reste X und Y ebenfalls Wasserstoff ist und der andere dieser beiden Reste eine Hydroxylgruppe, gebunden an den Benzolkern in der Stellung 5 oder der Stellung 7 des Indolgerüstes oder ein Sauerstoffatom mit daran gebundenem Glukoserest, ebenfalls in der Stellung 7 des Indolgerüstes darstellt.

Bezüglich der hier genannten substituierten Derivate der 2-Oxindol-3-essigsäure mit Hydroxylgruppen, bzw. über ein Sauerstoffatom an den Benzolkern gebundenen Glukoseresten sei auf die Veröffentlichung von P. Lewer, J. Chem. Soc. Perkin Trans. I, 1987, Seiten 753-757 verwiesen, sowie auf die Veröffentlichung von H.M. Nonhebel et al. in Plant Physiol., 1984, 76(4), Seiten 979-983 (siehe Chemical Abstracts, Vol. 102, Nr. 109920a). Bezüglich der chlor-, brom- und jodsubstituierten Derivate der 2-Oxo-1,2,3,4-tetrahydrochinolin-4-carbonsäure sei auf Beilstein, Hauptwerk, Band 22, Seite 308, bzw. Ergänzungswerk II, Band 22, Seiten 242 und 243, verwiesen.

Des weiteren ist diejenige 2-Oxo-1,2,3,4-tetrahydrochinolin-4-carbonsäure der Formel II bereits beschrieben, in welcher R, $R^1$, X und Y Wasserstoff bedeuten und Z für einen Ethylrest steht. Es sei in diesem Zusammenhang auf die Synthese dieser Chinolinderivate durch eine photochemische Ethylierung verwiesen, die von Ide, Akio et al. entwickelt wurde, und es sei in diesem Zusammenhang auf Chemical Abstracts, Vol. 88, Nr. 1, 2. Januar 1978, Columbus, Ohio, US; Abstract Nr. 6684q verwiesen, welches das Abstract der Originalarbeit in Bull. Che. Soc. JPN 1977, 50(8), 1959-63 darstellt.

Diejenige 2-Oxo-1,2,3,4-tetrahydrochinolin-4-carbonsäure der Formel II, in welcher R, Z, $R^1$ und X Wasserstoff bedeuten und Y einen Methylrest oder einen Methoxyrest in der 7-Stellung des Benzolkernes der Verbindungen der Formel II bedeuten, wurden durch photolytische Cyclisierung aus entsprechenden Carbonsäuren hergestellt, die statt des stickstoffhaltigen kondensierten heterocyclischen Restes der Tetrahydrochinolin-carbonsäure eine entsprechend ungesättigte aliphatische Kette aufwiesen, die über die Gruppe $NR^1$ an den Benzolkern gebunden ist. Dabei zeigte es sich, dass entsprechende Derivate, die einen Rest Y aufwiesen, welcher die Bedeutung von Wasserstoff, Chlor oder Nitro aufwies, nicht in analoger Weise durch Photocyclisierung hergestellt werden konnten. Es sei in diesem Zusammenhang auf die Veröffentlichung

von Kumar, Baldev et al. verwiesen, die in Chemical Abstracts, Vol. 90, Nr. 3, 15. Januar 1979, Columbus, Ohio, US; Abstract Nr. 21934f zusammengefasst ist, und die ein Abstract der Originalpublikation im Indian J, Chem., Sect. B 1978, 16B(8), 729-30 darstellt.

Von den Estern der 2-Oxindol-3-essigsäure der Formel I, bzw. der 2-Oxo-1,2,3,4-tetrahydrochinolin-4-carbonsäure der Formel II sind die Methylester bekannt, in welchen R Methyl ist und Z und $R^1$ Wasserstoff bedeuten und ferner X und Y beide für Wasserstoff oder für zwei Methoxygruppen in den Stellungen 5 und 6 des Benzolkernes des Indolgerüstes stehen oder X und Y gemeinsam eine Gruppierung der Formel

$-O-CH_2-O-$

ebenfalls in den Stellungen 5 und 6 des Indolgerüstes darstellen. Es sei auf die Veröffentlichung von S.J. McEvoy et al. in J. Org. Chem., Vol. 38, Nr. 19, 1973, Seiten 3350-3352, verwiesen, bzw. auf Beilstein, System Nr. 3366, Ergänzungswerk III/IV, Band 22, Seiten 3045 und 3046, Hauptwerk Band 22, Seiten 307 und 308.

Des weiteren ist derjenige Methylester der 2-Oxindol-3-essigsäure der Formel I bereits beschrieben, in welcher R Methyl ist, Z, X und Y Wasserstoff bedeuten und der Rest $R^1$ einen Methylsubstituenten bedeutet. Dieser Methylester der 1-Methyl-2-oxindol-3-essigsäure der Formel I wurde durch Cyclisierung unter Verwendung eines Organo-nickelkomplexes hergestellt, und es sei in diesem Zusammenhang auf die Veröffentlichung von Mori, Miwako et al. 'Reactions and syntheses ...' Chemical Abstracts, Vol. 85, Nr. 23, 6. Dezember 1976, Columbus, Ohio, US; Abstract Nr. 177179w verwiesen, die ein Abstract der entsprechenden Originalarbeit in Tetrahedron Lett. 1976, (21), 1807-1810 darstellt.

Auch der Methylester der unsubstituierten 2-Oxo-1,2,3,4-tetrahydrochinolin-4-carbonsäure, also diejenige Verbindung der Formel II, ist bereits in der Literatur beschrieben, in welcher R Methyl bedeutet und Z, $R^1$, X und Y Wasserstoff bedeuten. Es sei in diesem Zusammenhang auf die Veröffentlichung von J.A. Aeschlimann in Soc. 1926, Seiten 2902-2908 verwiesen.

Auch Ethylester der 2-Oxindol-3-essigsäure der Formel I, bzw. der 2-Oxo-1,2,3,4-tetrahydrochinolin-4-carbonsäure der Formel II sind bereits in der Literatur beschrieben.

So ist der Ethylester der unsubstituierten 2-oxindol-3-essigsäure, also die Verbindung der Formel I bekannt, in welcher R Ethyl ist und Z, $R^1$, X und Y für Wasserstoffatome stehen (siehe Beilstein, Ergänzungswerk III/IV, Band 22, Seite 3047).

Auch Ethylester der 2-Oxindol-3-essigsäure, in welchen der Rest $R^1$ eine Methylgruppe oder den Acetylrest darstellt, sind bereits in der Literatur beschrieben. Beispielsweise wurden die entsprechenden Verbindungen der Formel I, in welchen R für Ethyl steht, $R^1$ Methyl bedeutet, Z und X Wasserstoff bedeuten und Y entweder ebenfalls Wasserstoff oder Methoxy in der 5-Stellung des Benzolkerns der Indolessigsäure darstellt, als Ausgangsmaterial zur Herstellung einiger Indolalkaloide der Calabar-bohnen herangezogen. Es sei in diesem Zusammenhang auf die Veröffentlichung von Horne, Stephen et al., Chemical Abstracts, Vol. 116, Nr. 11, 16. März 1992, Columbus, Ohio, US; Abstract Nr. 106567u verwiesen, in welchem die Originalarbeit aus dem J. Chem. Soc., Perkin Trans. 1, 1991, (12) 2047-51 zusammengefasst ist.

Auch der Ethylester der unsubstituierten 2-Oxo-1,2,3,4-tetrahydro-chinolin-4-carbonsäure, also diejenige Verbindung der Formel II, in welcher R Ethyl ist, und Z, $R^1$, X und Y Wasserstoff bedeuten, ist bereits bekannt (siehe Beilstein, Ergänzungswerk III/IV, Band 22, Seiten 3045 und 3046).

Die 2-Oxindol-3-essigsäure der Formel I, bzw. die 2-Oxo-1,2,3,4-tetrahydrochinolin-4-carbonsäure der Formel II, in welcher R, Z, X und Y für Wasserstoffatome stehen und $R^1$ die Bedeutung eines Methylrestes, eines Phenylrestes, eines Benzylrestes, eines Acetylrestes oder des Acylrestes der p.-Chlorbenzoesäure, also des 4-Chlorbenzoylrestes aufweisen, sind ebenfalls bereits beschrieben (siehe Beilstein, System Nr. 3366, Ergänzungswerk II, Band 22, Seite 242, bzw. Hauptwerk, Band 22, Seite 308). Von den 2-Oxindol-3-essigsäuren der Formel I, in welchen $R^1$ ein Benzylrest ist, ist ferner der Methyl- und der Ethylester bekannt.

Ferner sind auch solche 2-Oxo-1,2,3,4-tetrahydrochinolin-4-carbonsäuren der Formel II bereits bekannt, in welchen Z, X und Y Wasserstoff ist und $R^1$ Phenyl bedeutet, und zwar sowohl die entsprechende freie Säure als auch der Methylester.

Auch solche 2-Oxo-1,2,3,4-tetrahydrochinolin-4-carbonsäuren der Formel II sind bekannt, in welchen Z, X und Y Wasserstoff ist und $R^1$ Butyl bedeutet, und zwar die entsprechende freie Säure, der Butylester und der Ester eines speziellen Bicycloalkanols.

Keines dieser bereits bekannten Derivate der Formel I, bzw. II wurde in irgendeinem Arzneimittel als Wirkstoff eingesetzt.

Hingegen sind verschiedene Säureamide der 2-Oxo-1,2,3,4-tetrahydrochinolin-carbonsäure, in welchen statt der Gruppe der Formel -COOR (siehe Formel II) ein Säureamidrest der Formel -CON $(R_3)_2$ vorliegt, wobei der Rest N $(R_3)_2$ von substituiertem Piperazinyl stammt, bezüglich ihrer Wirkung als positiv inotrope Mittel, bzw. ihrer Wirkung der Beeinflussung des Herzrhythmus untersucht worden, wobei die Säureamidgruppe der angegeben Struktur sich entweder in der 4-Stellung oder in anderen Stellungen am heterocyclischen Kern oder im Benzolkern des 2-Oxo-1,2,3,4-tetrahydrochinolin-derivates befand. Dabei zeigte es sich, dass nur diejenigen Verbindungen eine entsprechende Wirkung zeigten, in denen sich die Säureamidgruppe in der 6-Stellung, also an den Benzolkern gebunden befindet, während die entsprechenden Verbindungen mit einer Säureamidgruppe in der 4-Stellung (siehe Formel II) der Tetrahydrochinolinstruktur unwirksam waren. Es sei in diesem Zusammenhang auf die Veröffentlichung von M. Tominaga et al. in Chem. Pharm. Bull., Vol. 34 (1986), Seiten 682-689, insbesondere die Tabelle V auf Seite 689, verwiesen.

## BESCHREIBUNG DER ERFINDUNG

Ein Gegenstand der vorliegenden Erfindung ist ein pharmazeutisches Präparat zur Modulierung der Immunreaktion warmblütiger Tiere, das dadurch gekennzeichnet ist, dass es als Wirkstoff ein Derivat der 2-Oxindol-3-essigsäure der Formel I, oder ein entsprechendes Derivat der 2-Oxo-1,2,3,4-tetrahydrochinolin-4-carbonsäure der Formel II

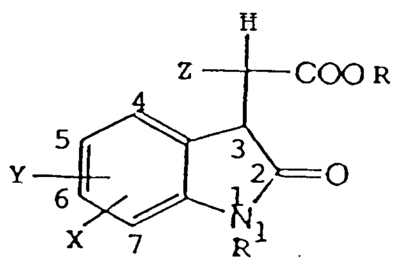

I

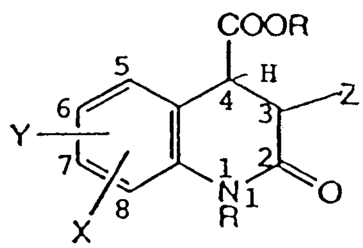

II

enthält, wobei die Verbindung der Formel I, gegebenenfalls mit der Verbindung der Formel II im Gleichgewicht steht und

$R^1$ ein Wasserstoffatom, einen Alkylrest mit 1-6 Kohlenstoffatomen, einen Aralkylrest mit 1-6 Kohlenstoffatomen im Alkylteil, einen Cycloalkylrest oder einen Acylrest der Formel

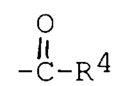

darstellt, in welchem

$R^4$ ein Wasserstoffatom, ein Alkylrest mit 1-6 Kohlenstoffatomen, ein Aralkylrest mit 1-6 Kohlenstoffatomen im Alkylteil, ein Arylrest oder ein Cycloalkylrest ist,

Z ein Wasserstoffatom, ein Alkylrest mit 1-6 Kohlenstoffatomen, ein Aralkylrest mit 1-6 Kohlenstoffatomen in der Alkylgruppe, ein Cycloalkylrest oder ein Arylrest ist, und

X und Y unabhängig voneinander für Wasserstoffatome, Alkylreste, Cycloalkylreste, Arylreste, Hydroxygruppen, Halogenatome, Nitrogruppen, Ethergruppierungen der Formel

$-O-R^2$,

Estergruppierungen der Formel

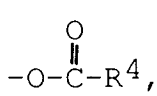

oder Estergruppierungen der Formel

$$-\underset{\underset{O}{\|}}{C}-O-R^2$$

stehen,

wobei in den oben angegebenen Formeln der Rest

$R^4$ die oben angegebene Bedeutung besitzt und der Rest

$R^2$ ein Alkylrest mit 1-6 Kohlenstoffatomen, ein Aralkylrest mit 1-6 Kohlenstoffatomen im Alkylteil, ein Cycloalkylrest oder ein Arylrest ist oder die Reste X und Y gemeinsam eine Gruppierung der Formel

$-O-(CH_2)_n-O-$

darstellen, in welcher

n einen Wert von 1-4 aufweist und R ein Wasserstoffatom, ein Alkylrest mit 1-6 Kohlenstoffatomen, ein Aralkylrest mit 1-6 Kohlenstoffatomen im Alkylteil, ein Cycloalkylrest oder ein Arylrest ist oder der Rest R einen esterartig gebundenen Hexoserest oder eine Kette aus 2-6 Hexoseresten darstellt, wobei der Hexoserest, bzw. ein oder mehrere der Hexosereste, gegebenenfalls ausserdem Substituenten tragen, wie beispielsweise Hydroxylgruppen dieser Zuckerreste, die acyliert sind, und

wobei der Wirkstoff der Formel I, bzw. II, einschliesslich aller oben definierten Derivate in freier Form oder Form von pharmazeutisch annehmbaren Salzen vorliegen, oder wobei der Wirkstoff eine Mischung darstellt, die mindestens zwei der oben definierten Derivate oder deren pharmazeutisch annehmbare Salze enthält.

Wie aus der chemischen Struktur der 2-Oxindol-3-essigsäurederivate der Formel I ersichtlich ist, weisen diejenigen Verbindungen, in denen Z ein Wasserstoffatom darstellt, ein chirales Zentrum in der 3-Stellung des Indolringes auf, und diejenigen Verbindungen, in welchen Z einer der angeführten Kohlenwasserstoffreste ist, ein weiteres chirales Zentrum auf, nämlich das Kohlenstoffatom, an welches dieser Rest Z gebunden ist.

Des weiteren weisen auch diejenigen 2-Oxo-1,2,3,4-tetrahydrochinolin-4-carbonsäuren der Formel II, in welchen Z ein Wasserstoffatom ist, ein chirales Zentrum in der Stellung 4 des Tetrahydrochinolingerüstes auf, und diejenigen Verbindungen der Formel II, in denen Z einer der angeführten Kohlenwasserstoffreste ist, besitzen ein weiteres chirales Zentrum, nämlich das Kohlenstoffatom in der Stellung 3 des Tetrahydrochinolingerüstes.

In der Literatur sind ausschliesslich Racemate der 2-Oxindol-3-essigsäure und der Derivate derselben der Formel I und auch der 2-Oxo-1,2,3,4-tetrahydrochinolin-4-carbonsäure und der Derivate derselben der Formel II beschrieben.

Jetzt wurde erstmals die Auftrennung der unsubstituierten der 2-Oxo-1,2,3,4-tetrahydrochinolin-4-carbonsäure der Formel II durchgeführt, also desjenigen Derivates der Formel II, in welchem sämtliche Reste Z, R, $R^1$, X und Y Wasserstoff sind.

Aus dem Racemat der unsubstituierten 2-Oxo-1,2,3,4-tetrahydro-chinolin-4-carbonsäure der Formel II wurden die beiden Enantiomeren in reiner Form isoliert. Diese optisch aktiven Antipoden stellen neue chemische Verbindungen dar.

Ein weiterer Gegenstand der vorliegenden Erfindung sind daher solche neuen Verbindungen der Formel II, die dadurch gekennzeichnet sind, dass sie die folgende Formel IIb

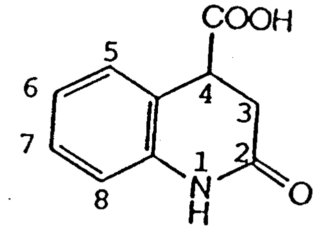

IIb

aufweisen, und dass sie in Form der optisch aktiven Enantiomeren vorliegen.

In den erfindungsgemässen pharmazeutischen Präparaten kann als Wirkstoff ein Racemat der Verbindungen der Formel I bzw. II oder eine an einem der beiden optischen Antipoden angereicherte Form oder die Reinform des Antipoden der Verbindung der Formel I oder II enthalten sein.

Bevorzugte Wirkstoffe, welche in den erfindungsgemässen pharmazeutischen Präparaten enthalten sind, sind diejenigen Verbindungen der Formel I, bzw. II, in welchen

Z und $R^1$ ein Wasserstoffatom bedeuten,

R ein Wasserstoffatom, einen Alkylrest mit 1-6 Kohlenstoffatomen, einen Cycloalkylrest mit 3-8 Kohlenstoffatomen oder einen Arylrest, vorzugsweise einen Phenylrest oder einen substituierten Phenylrest bedeutet und

X und Y unabhängig voneinander für Wasserstoffatome, Hydroxygruppen, Halogenatome, Nitrogruppen oder Ethergruppierungen der Formel

$-O-R^2$

oder Estergruppierungen der Formel

$$-O-\overset{O}{\overset{\|}{C}}-R^4,$$

stehen, wobei in diesen Gruppierungen der Rest

$R^2$ einen Alkylrest mit 1-6 Kohlenstoffatomen oder einen Arylrest, vorzugsweise einen unsubstituierten oder substituierten Phenylrest darstellt und

$R^4$ ein Alkylrest mit 1-6 Kohlenstoffatomen, ein Arylrest, vorzugsweise ein unsubstituierter oder substituierter Phenylrest oder ein Cycloalkylrest mit 3-7 Kohlenstoffatomen ist.

Speziell bevorzugte Wirkstoffe sind diejenigen Verbindungen der Formel I, bzw. II, in welchen sämtliche Reste R, Z, X, Y und $R^1$ die Bedeutung von Wasserstoffatomen aufweisen.

Die in den erfindungsgemässen pharmazeutischen Präparaten enthaltenen Wirkstoffe der Formeln I und II umfassen sowohl neue chemische Verbindungen, als auch solche chemische Verbindungen, die in der Literatur bereits beschrieben sind, die jedoch bisher noch nie in irgendeinem pharmazeutischen Präparat als Wirkstoff eingesetzt wurden.

Die bereits bekannten Wirkstoffe der Formel I, bzw. II wurden nach Arbeitsverfahren hergestellt, die in der Literatur bereits beschrieben sind. So ist es beispielsweise möglich, die 2-Oxindol-3-essigsäure durch Oxydation von Indol-3-essigsäure nach demjenigen Arbeitsverfahren herzustellen, das von K. Szabo-Pisztay, L. Szabo, in Synthesis 1979, 176 beschrieben ist.

Die Derivate der 2-Oxindol-3-essigsäure können beispielsweise aus der unsubstituierten Verbindung durch entsprechende Substitutionsreaktionen, beispielsweise Halogenierungen, Nitrierungen, Veresterungen und ähnliche an sich bekannte Arbeitsverfahren hergestellt werden.

Auch ist es möglich, durch Verwendung entsprechend substituierter Ausgangsmaterialien, die in gewünschter Weise substituierten Produkte nach an sich bekannten Syntheseverfahren herzustellen.

Des weiteren ist es möglich, die unsubstituierte 2-Oxindol-3-essigsäure durch Behandlung mit zweinormaler Salzsäure in die unsubstituierte 2-Oxo-1,2,3,4-tetrahydrochinolin-4-carbonsäure umzuwandeln (siehe das Gleichgewicht zwischen der Verbindung der Formel Ia und der Formel IIa).

Ferner ist es möglich, beispielsweise nach dem Verfahren, das von R.C. Elderfield, H.H. Rembges, in J. Org. Chem. 32 /1967, Seite 3809 beschrieben ist, die Essigsäureseitenkette der Formel

$$-\overset{Z}{\overset{/}{C}}H-COOR$$

in ein entsprechendes Indolderivat einzuführen. Es sei in diesem Zusammenhang auch auf die Syntheseverfahren hingewiesen, die von P.L. Julian et al. im J. Am. Chem. Soc., Band 75, 1953, Seiten 5305 - 5309, und von J.A. Aeschlimann, in J. Chem. Soc., 1926, Seiten 2902 - 2912 beschrieben sind.

Aus der unsubstituierten 2-Oxo-1,2,3,4-tetrahydro-chinolin-4-carbonsäureder Formel II können neue Derivate dieser Säure durch entsprechende Substitutionsreaktionen und weitere Arbeitsschritte hergestellt

werden.

Sowohl die bereits bekannten als auch die neuen Wirkstoffe der Formeln I, bzw. II, in welchen die Substituenten R, $R^1$, Z, X und Y die oben angegebene Bedeutung aufwiesen, besassen die überraschend hohe Wirksamkeit bezüglich der Modulierung der Immunreaktion.

Ein weiterer Gegenstand der vorliegenden Erfindung sind neue chemische Verbindungen der Formeln I und II.

Eine Klasse an diesen neuen chemischen Verbindungen sind solche der Formeln I und II

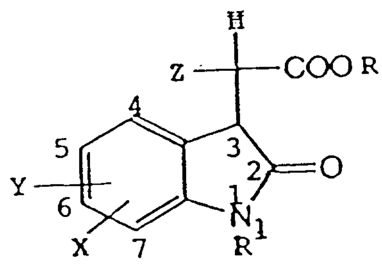

I

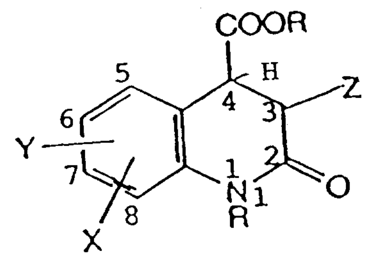

II

in welchen

$R^1$ ein Wasserstoffatom, einen Alkylrest mit 1-6 Kohlenstoffatomen, einen Aralkylrest mit 1-6 Kohlenstoffatomen im Alkylteil, einen Cycloalkylrest oder einen Acylrest der Formel

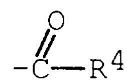

darstellt, in welchem

$R^4$ ein Wasserstoffatom, ein Alkylrest mit 1-6 Kohlenstoffatomen, ein Aralkylrest mit 1-6 Kohlenstoffatomen im Alkylteil, ein Arylrest oder ein Cycloalkylrest ist,

Z ein Wasserstoffatom, ein Alkylrest mit 1-6 Kohlenstoffatomen, ein Aralkylrest mit 1-6 Kohlenstoffatomen in der Alkylgruppe, ein Cycloalkylrest oder ein Arylrest ist, und

X und Y unabhängig voneinander für Wasserstoffatome, Alkylreste, Cycloalkylreste, Arylreste, Hydroxygruppen, Halogenatome, Nitrogruppen, Ethergruppierungen der Formel

$-O-R^2$,

Estergruppierungen der Formel

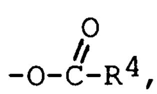

Estergruppierungen der Formel

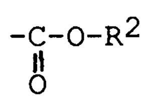

stehen, und wobei in den oben angegebenen Formeln der Rest

$R^4$ die oben angegebene Bedeutung besitzt und der Rest

$R^2$ ein Alkylrest mit 1-6 Kohlenstoffatomen, ein Aralkylrest mit 1-6 Kohlenstoffatomen im Alkylteil, ein Cycloalkylrest oder ein Arylrest ist oder die Reste X und Y gemeinsam eine Gruppierung der Formel

$-O-(CH_2)_n-O-$

darstellen, in welcher

n einen Wert von 1-4 aufweist und

R ein Wasserstoffatom, ein Alkylrest mit 1-6 Kohlenstoffatomen, ein Aralkylrest mit 1-6 Kohlenstoffatomen im Alkylteil, ein Cycloalkylrest oder ein Arylrest ist oder einen esterartig gebundenen Hexoserest oder eine Kette aus 2-6 Hexoseresten darstellt, wobei der Hexoserest, bzw. ein oder mehrere der Hexosereste gegebenenfalls ausserdem Substituenten tragen, wie beispielsweise Hydroxylgruppen dieser Zuckerreste, die acyliert sind,

unter der Voraussetzung, dass in denjenigen Verbindungen der Formel I bzw. II, in welchen

R ein Wasserstoffatom oder ein Methylrest oder ein Ethylrest ist, und ferner

$R^1$ Wasserstoff oder Methyl bedeutet und ausserdem

Z Wasserstoff ist,

die Reste X und Y nicht gemeinsam zwei Wasserstoffatome, zwei Chloratome, zwei Bromatome, zwei Methoxygruppen oder die Gruppe der Formel $-O-CH_2-O-$ bedeuten dürfen, und ausserdem dann, wenn der Rest X Wasserstoff ist, der Rest Y eine andere Bedeutung aufweisen muss als diejenige eines Jodatoms, einer Hydroxygruppe, einer Methoxygruppe oder einer Methylgruppe und

unter der weiteren Voraussetzung, dass in denjenigen Verbindungen der Formel I oder II, in welchen Z, X und Y ein Wasserstoffatom bedeuten, R Wasserstoff, Methyl, Ethyl oder Butyl ist, der Rest

$R^1$ eine andere Bedeutung aufweisen muss als diejenige eines Methylrestes, eines Butylrestes, eines Benzylrestes, eines Acetylrestes oder eines 4-Chlorbenzoylrestes und

unter der weiteren Voraussetzung, dass in denjenigen 2-Oxo-1,2,3,4-tetrahydrochinolin-4-carbonsäuren der Formel II, in welchen Z ein Ethylrest ist, mindestens einer der Reste R, $R^1$, X und Y eine andere Bedeutung aufweisen muss als diejenige eines Wasserstoffatomes,

sowie Salze, dieser neuen Wirkstoffe der Formeln I, bzw. II.

Bevorzugte neue chemische Verbindungen der Formel I, bzw. II, sind die entsprechenden Ester der angegebenen Formeln, in denen nämlich

R ein Alkylrest mit 3-6 Kohlenstoffatomen, ein Aralkylrest mit 1-6 Kohlenstoffatomen im Alkylteil, in welchem der Arylrest ein Phenylrest oder ein substituierter Phenylrest ist, ein Cycloalkylrest mit 3-7 Kohlenstoffatomen, ein Arylrest, vorzugsweise ein substituierter oder unsubstituierter Phenylrest ist, oder R einen esterartig gebundenen Hexoserest oder eine Gruppe aus 2-6 Hexoseresten, beispielsweise Glucosereste, wobei diese Hexosereste gegebenenfalls noch substituiert sind, darstellt, und wobei

die Reste $R^1$, Z, X und Y die oben angegebene Bedeutung besitzen, wobei jedoch

dann, wenn $R^1$ ein Butylrest ist und Z, X und Y für Wasserstoff stehen, der Rest R eine andere Bedeutung aufweisen muss als diejenige eines Butylrestes,

und wobei diese Verbindungen der Formel I oder II in freier Form oder in Form von Salzen vorliegen.

Im Falle, dass in diesen Estern der Formel I, bzw. II der Rest R einen esterartig gebundenen Hexoserest oder eine Gruppe aus 2-6 Hexoseresten darstellt, können diese Zuckerreste gegebenenfalls noch weitere Substituenten tragen, die an diese gebunden sind, nämlich vorzugsweise über Hydroxylgruppen der entsprechenden Hexosereste. Es ist auch möglich, dass an einen derartigen esterartig gebundenen Hexoserest ein Rest eines Aglycons gebunden ist oder dass eine Kette aus 2-6 Hexoseresten durch den Rest eines Aglycons unterbrochen ist. Als Beispiele seien Aglycone genannt, die durch Etherbindungen oder Esterbindungen an Hydroxylgruppen der Hexosereste gebunden sind.

Eine bevorzugte Gruppe an Substituenten der Hexosereste sind solche, die über eine Etherbindung an die Grundstruktur des Hexoserestes gebunden sind, also entsprechende Hexosereste, in welchen statt ein oder mehrerer der Hydroxygruppen entsprechende Gruppierungen der Formel $-OR^6$ vorliegen, in welchen $R^6$ einen etherartig gebundenen organischen Rest darstellt, beispielsweise einen Phenylrest oder einen niederen Alkylrest oder in welchen ein entsprechender etherartig gebundener Rest einen zweiwertigen organischen Rest darstellt, der zwei unterschiedliche Hydroxylgruppen eines Hexoserestes oder von zwei unterschiedlichen Hexoseresten miteinander verbindet, beispielsweise unter Ausbildung der Struktur eines cyclischen Diethers.

Eine weitere bevorzugte Klasse an Substituenten, die an entsprechende Hexosereste gebunden sein können, sind solche Hexosereste, in welchen ein oder mehrere der Hydroxylgruppen acyliert sind, insbesondere durch Acylreste, welche sich von aromatischen, alicyclischen oder aliphatischen Carbonsäuren ableiten, wie zum Beispiel Ameisensäure, Essigsäure oder Propionsäure. Auch in diesem Falle können gegebenenfalls zwei unterschiedliche an Hexosereste gebundene Hydroxylgruppen miteinander durch den Acylrest einer zweiwertigen Säure verbunden sein, und beispielsweise können zwei Hydroxylgruppen durch Acylierung mit einem aktiven Kohlensäurederivat in eine Struktur der Formel

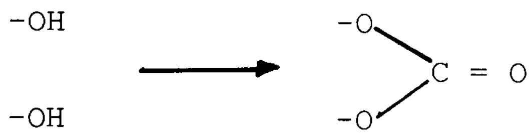

oder durch Acylierung mit einer Dicarbonsäure in eine Struktur der Formel

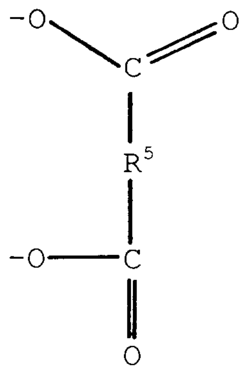

umgewandelt sein. Für den Fall, dass diese Dicarbonsäure Maleinsäure ist, steht $R^5$ für die Gruppe $-CH_2-$.

Eine weitere bevorzugte Gruppe an neuen chemischen Verbindungen der Formel I, bzw. II, sind die entsprechenden freien Carbonsäuren, also diejenigen Verbindungen der Formel I, bzw. II, in welchen

R Wasserstoff bedeutet und ferner

Z ein Alkylrest mit 1-6 Kohlenstoffatomen, ein Aralkylrest mit 1-6 Kohlenstoffatomen in der Alkylgruppe, in welchem der Arylrest vorzugsweise ein unsubstituierter oder substituierter Phenylrest ist, ein Cycloalkylrest mit 3-7 Kohlenstoffatomen oder ein Arylrest, vorzugsweise ein substituierter oder unsubstituierter Phenylrest ist und

X, Y und $R^1$ die weiter vorne angegebenen Bedeutungen besitzen,

unter der Voraussetzung, dass dann, wenn in diesen Säuren Z ein Ethylrest ist, mindestens einer der Substituenten $R^1$, X und Y eine andere Bedeutung aufweisen muss als diejenige eines Wasserstoffatomes und

wobei diese Verbindungen der Formel I, bzw. II in freier Form oder in Form von Salzen vorliegen.

Eine weitere bevorzugte Klasse an erfindungsgemässen Verbindungen der Formel I, bzw. II sind diejenigen, in welchen

die Reste R, Z und $R^1$ die weiter vorne angegebene Bedeutung besitzen und mindestens einer der Reste X und Y die Bedeutung von Alkylresten mit mindestens 2 Kohlenstoffatomen, Cycloalkylresten, Phenylresten, Nitrogruppen, Alkoxyreste mit mindestens 2 Kohlenstoffatomen, oder Estergruppierungen der Formel

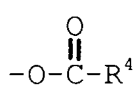

aufweist, wobei in dieser Gruppierung

$R^4$ ein Alkylrest mit 1-6 Kohlenstoffatomen, ein Arylrest, vorzugsweise ein unsubstituierter oder substituierter Phenylrest oder ein Cycloalkylrest mit 3-7 Kohlenstoffatomen ist,

wobei jedoch dann, wenn mindestens einer der Reste R, Z oder $R^1$ eine andere Bedeutung aufweist als diejenige eines Wasserstoffatoms, der Rest X und/oder der Rest Y ausserdem die Bedeutung von Methylresten und/oder Methoxyresten aufweisen kann, wobei jedoch dann, wenn in den Verbindungen der Formel I Z und $R^1$ Wasserstoff bedeuten und R Methyl ist und beide Reste X und Y für Methoxygruppen stehen, sich diese nicht in den Stellungen 5 und 6 des Benzolkernes des Indolgerüstes der Verbindungen der Formel I befinden dürfen.

Bezüglich der pharmazeutischen Wirksamkeit der in dem pharmazeutischen Präparat enthaltenen Wirkstoffe wurde festgestellt, dass diese eine Modulierung der Immunreaktion bei warmblütigen Tieren hervorrufen, indem sie die Bildung von Lymphokininen stimulieren und durch diese Stimulierung kann

beispielsweise die Bildung von Interferon angeregt und/oder die Bildung von TNF-Faktoren (Tumor-Nektrosefaktoren) angeregt werden.

Es zeigte sich ferner, dass die in den erfindungsgemässen pharmazeutischen Präparaten enthaltenen Wirkstoffe bezüglich ihrer Wirksamkeit gehemmt werden, wenn zusätzlich solche Faktoren anwesend sind, die zu einer Hemmung der Stimulierung der Makrophagen oder zu einer Hemmung der Stimulierung der T-Zellen führen.

Die Modulierung der Immunreaktion durch die erfindungsgemässen pharmazeutischen Präparate wurden an Mäusen nach dem PCT-Test getestet. Dabei zeigte es sich, dass eine deutliche Wirksamkeit schon dann erzielt werden konnte, wenn die entsprechenden Präparate an die Tiere in einer Dosierung von nur 0,02 bis 0,06 mg/kg Körpergewicht des Tieres pro Tag verabreicht wurden.

Durch die Verabreichung der erfindungsgemässen pharmazeutischen Präparate konnte die Lebensdauer von Mäusen, denen verschiedene Krebszellen (z.B. Prostatakrebs, Brustkrebs, usw.) eingepflanzt worden waren, im Vergleich zur Gruppe ohne Verabreichung der entsprechenden Präparate, wesentlich verlängert werden.

Die erfindungsgemässen pharmazeutischen Präparate, welche die bekannten oder neuen Wirkstoffe der Formel I oder II enthalten, können beispielsweise verwendet werden, um Tumore, verschiedene Viruskrankheiten, z.B. Aids, und andere krankhafte Zustände zu behandeln, die mit einer reduzierten Immunreaktion verbunden sind.

Einige Ausführungsarten der Erfindung seien anhand der nachfolgenden Beispiele näher erläutert:

## Beispiel 1

### Herstellung der 2-Oxindol-3-essigsäure

Die im Titel genannte Verbindung entspricht der Formel I, wobei sämtliche Substituenten R, $R^1$, Z, Y und X Wasserstoff sind. Die Herstellung dieser Verbindung erfolgte nach dem Verfahren, das von K. Szabo-Pisztay, L. Szabo in Synthesis 1979, 276 beschrieben ist.

Zu einer Lösung von 20 g (1,14 mmol) an Indol-3-essigsäure in 8,1 ml (1,14 mmol) an Dimethylsulfoxid setzte man langsam 19,3 ml (228 mmol) an konzentrierter Salzsäure zu. Die Reaktion verlief stark exotherm, wobei das Dimethylsulfoxid zu Dimethylsulfid reduziert wurde. Das Reaktionsgemisch verfärbte sich dunkelviolett, und es bildete sich eine grosse Menge an Niederschlag. Die Reaktion wurde vier Stunden lang bei Zimmertemperatur belassen und man verdünnte anschliessend mit 20 ml Wasser, stellte den pH-Wert durch Zugabe einer konzentrierten wässrigen Ammoniaklösung auf einen Wert im Bereich von 5 bis 6 ein und extrahierte dann dreimal mit 200 ml Essigsäure-ethylester. Die abgetrennten organischen Phasen wurden vereinigt und einmal mit 200 ml Wasser gewaschen, anschliessend über wasserfreiem Magnesiumsulphat getrocknet und unter Vakuum eingedampft. Der Rückstand wurde in warmem Wasser gelost, filtriert und das Wasser unter Vakuum abgedampft.

Beim Umkristallisieren aus einer Mischung von Aceton + Hexan erhielt man 7,5 g an gelblichen Kristallen, entsprechend einer Ausbeute von 34% der theoretischen Ausbeute.

Das Produkt besass einen Schmelzpunkt von 140 bis 142° C, der somit mit den in der Literatur angegebenen Werten übereinstimmte.

Im kernmagnetischen Resonanzspektrum erhielt man die folgenden Signale:

$^1$H-NMR(90 MHz, Py-$D_5$): 3.43 (dxd, $J_1$ = 9, $J_2$ = 15, 1H, H-C(1')), 3.83 (dxd, $J_2$ = 15, $J_3$ = 4.5, 1H, H-C(1')), 4.46 (dxd, $J_1$ = 9, $J_3$ = 4.5, 1H, H-C(3)), 7.2-7.6 (m, 3H, arom.), 7.86 (d, J = 7.5, 1H, arom.).

## Beispiel 2

### Herstellung von 2-Oxo-1,2,3,4-tetrahydrochinolin-4-carbonsäure

Die im Titel genannte Verbindung entspricht der Formel II, wobei sämtliche Reste R, $R^1$, Z, X und Y die Bedeutung von Wasserstoffatomen besassen.

Die Herstellung erfolgte sowohl nach dem von R.C. Elderfield, H.H. Rembges in J. Org. Chem. 32 (1967), 3809 beschriebenen Verfahren, als auch durch Umlagerung der gemäss Beispiel 1 hergestellten 2-Oxindol-3-essigsäure unter Bildung der im Titel genannten Säure.

Zu diesem Zwecke erwärmte man eine Suspension von 3 g (15,7 mmol) der nach Beispiel 1 erhaltenen 2-Oxindol-3-essigsäure in 150 ml an 2 normaler Salzsäure auf 100° C und behielt diese Temperatur während 2 1/2 Stunden. Nach dem Abziehen des Lösungsmittels wurde der feste Rückstand aus Methanol umkristallisiert und im Hochvakuum getrocknet.

Dabei erhielt man 1,63 g an farblosen Kristallen, die sich bei der Dünnschichtchromatographie (Kieselgel, MeOH/EtOAc 1:3 + 1 Tropfen AcOH) als reines Produkt erwiesen.

Dieses Produkt besass einen Schmelzpunkt von 219-221° C und dieser Schmelzpunkt war identisch mit demjenigen Produkt, das nach dem in der Literatur beschriebenen Verfahren hergestellt wurde, und auch der Mischschmelzpunkt der beiden Produkte war identisch.

Im kernmagnetischen Resonanzspektrum erhielt man die folgenden Signale:

$^1$H-NMR (90 MHz, Py-$D_5$): 3.26 (dxd, $J_1$ = 7.5, $J_2$ = 16.5, 1H, H-C(3)), 3.72 (dxd, $J_2$ = 16.5, $J_3$ = 4, 1H, H-C(3)), 4.53 (dxd, $J_1$ = 7.5, $J_3$ = 4, 1H, H-C(4)), 7.2-7.5 (m, 3H, arom.), 7.93 (d, J = 7.5, 1H, arom.), 11.6(s br., 1H, H-N).

Beispiel 3

Durchführung von pharmakologischen Tests mit den gemäss Beispiel 1 und Beispiel 2 hergestellten Verbindungen

Zur Bestimmung der biologischen Aktivität wurde der PCV-Test durchgeführt. Die Bezeichnung PCV basiert auf der englischsprachigen Bezeichnung Packed Cell Volume.

Schweizer Mäuse wurden bei der NCI (Cr:SW) käuflich erworben und für den Test verwendet, sobald die weiblichen Tiere 5 - 7 Wochen alt waren und ein Gewicht von 19-22 g aufwiesen.

Am Tage 0 wurden an jedes Tier 1,5 x $10^5$ Tumor S180 Zellen durch intraperitoneale Injektion verabreicht.

Am Tage 1 wurden die Tiere nach dem Zufallsprinzip in einzelne Gruppen sortiert, wobei jede Gruppe 6 Mäuse umfasste und in jeder Gruppe die Tiere ein ähnliches Gewicht aufwiesen. Das Gewicht sämtlicher Tiere der Gruppe wurde vermerkt.

In diesem Test wurde sowohl die gemäss Beispiel 2 hergestellte unsubstituierte 2-Oxo-1,2,3,4-tetrahydro-chinolin-4-carbonsäure der Formel II als auch die gemäss Beispiel 1 hergestellte, unsubstituierte 2-Oxindol-3-essigsäure getestet.

Die Säure der Formel II, bzw. der Formel I, wurde in einer Phosphatpufferlösung eines pH-Wertes von 7 (abgekürzt als PBS) gelöst. In manchen Fällen war es nötig, eine sehr geringe Menge an Natriumbicarbonat zuzugeben, um durch eine Erhöhung des pH-Wertes von 7 auf 7,5 eine vollständige Lösung zu erreichen. Die Verabreichung der PBS-Lösungen erfolgte durch intraperitoneale Injektion, die täglich, und zwar beginnend vom 1. Tag bis zum 5. Tag, sodass pro Tier insgesamt 5 Injektionen verabreicht wurden.

Jedes Tier erhielt 0,2 ml an PBS-Lösungen unterschiedlicher Konzentration um die in der nachfolgenden Tabelle angegebene täglich Dosierung pro Maus zu erreichen. An die Mäuse der Gruppe zu Vergleichszwecken wurden nur 0,2 ml an PBS, ohne jeglichen Wirkstoff, verabreicht. Die PBS-Lösungen der Verbindung der Formel II, bzw. der Verbindung der Formel I, wurde täglich hergestellt und jeweils auf die gewünschte Konzentration verdünnt.

Am 7. Tag oder am 8. Tag zeigten die Mäuse der Kontrollgruppe sichtbar geschwollene Abdomina. Die Auswertung des Tests erfolgte dementsprechend am 7. oder am 8. Tag.

Durchführung der Auswertung:

Schritt (a): Sämtliche Mäuse jeder Gruppe wurden einzeln gewogen und getötet. Ein Mikrokapilarröhrchen wurde mit der in der freien Bauchhöhle befindlichen Flüssigkeit (Ascites-Flüssigkeit) gefüllt, indem man einen Schnitt in die Bauchwand jeder Maus durchführte.

Jede der Proben wurde 7 Min. lang bei 10'000 UpM zentrifugiert (internationale Cytocrit-Zentrifuge). Die Prozente des Zellvolumens (Vc) jeder Probe wurden gemessen.

Schritt (b): Die Flüssigkeit der Bauchhöhle jedes Tieres wurde durch Drainage entfernt, und die geöffnete Bauchhöhle auf absorbierender Gaze getrocknet. Die Mäuse wurden nochmals gewogen, und der Unterschied des Körpergewichtes stellte die Volumina der in der Bauchhöhle befindlichen Flüssigkeit (Volumen Va) jeder Maus dar. In diesem Falle wurde angenommen, dass die Körperflüssigkeit eine spezifische Masse von 1,0 besass.

Schritt (c): Berechnung des PCV:

Das PCV wird nach der folgenden Formel berechnet: PCV = %Vc x Va ml.

Das durchschnittliche PCV für jede Gruppe der durch Verabreichung unterschiedlicher Mengen der Verbinung der Formel II, bzw. I behandelten Tiere (Gruppen T) und das PCV der Tiere der Gruppe zu Vergleichszwecken (C) wurde berechnet und daraus wurde ferner für jede der behandelten Gruppen der Wert T/C berechnet.

Ein Wert T/C von 0,5 zeigt dementsprechend an, dass ein 50%-iges Wachstum der Zellen stattgefunden hat, verglichen mit dem Zellwachstum in der nicht behandelten Gruppe zu Vergleichszwecken.

Die Ergebnisse dieser Tests sind in der folgenden Tabelle zusammengestellt.

Bei den angegebenen Werten handelt es sich um den Durchschnittswert von zwei Versuchen, die jeweils mit 6 oder 7 Mäusen pro Gruppe durchgeführt wurden. In der ersten Spalte ist die Nummer der Gruppe angegeben, wobei die Gruppe 1 diejenige zu Vergleichszwecken ist, bei der also an die Tiere nur jeweils 0,2 ml an PBS verabreicht wurden, ohne jeglichen Wirkstoff.

In den Gruppen 2 - 7 wurde PBS verabreicht, welches die angegebenen Mengen an der unsubstituierten 2-Oxindol-3-essigsäure der Formel I enthielten, und in den Gruppen 8 - 13 wurde jeweils 0,2 ml an PBS injiziert, welche die angegebenen Mengen an der unsubstituierten Oxo-1,2,3,4-tetrahydro-chinolin-4-carbonsäure der Formel II enthielten. Im zuletzt genannten Fall wurde der pH-Wert der Lösung durch Zugabe von Natriumbicarbonat auf 7,5 eingestellt, um eine vollständige Auflösung der Säure der Formel II zu erreichen.

Insgesamt wurde in der Gruppe zu Vergleichszwecken zweimal je 7 Tiere, also insgesamt 14 Tiere getestet, in den Gruppen 2 - 13 je Versuch 6 Tiere, also pro Gruppe insgesamt 12 Tiere getestet.

Sämtliche Tiere wurden am 7. Tage getötet und den vorher beschriebenen Versuchen unterworfen. Aus diesem Grunde hatte sich auch in der Gruppe zu Vergleichszwecken der Tumor S180 bei keinem Tier so weit entwickelt, dass ein natürlicher Tod eintrat. Somit hatten in jeder der getesteten Gruppe bis zum 7. Tag sämtliche getesteten Tiere überlebt.

In der dritten Spalte der folgenden Tabelle wird für jede Gruppe der getesteten Tiere die durchschnittliche Gewichtzunahme in g der Tiere in jeder der Testgruppen 1 - 13 vom Testbeginn (Tag 0) bis zum Tag 7 (Tag der Tötung) angegeben.

| Gruppe Nr. | Dosierung in mg/Maus | Gewichtszunahme in g | PCV (μl) | T/C |
|---|---|---|---|---|
| 1 | 0 | +2.4 | 470 | 1.0 |
| 2 | 0.02 | +2.0 | 0 | 0.0 |
| 3 | 0.01 | +2.0 | 0 | 0.0 |
| 4 | 0.005 | +2.2 | 0 | 0.0 |
| 5 | 0.0025 | +1.7 | 0 | 0.0 |
| 6 | 0.00125 | +1.4 | 75 | 0.16 |
| 7 | 0.000625 | +2.5 | 169 | 0.36 |
| 8 | 0.02 | +2.2 | 0 | 0.0 |
| 9 | 0.01 | +2.0 | 0 | 0.0 |
| 10 | 0.005 | +1.5 | 0 | 0.0 |
| 11 | 0.0025 | +1.6 | 0 | 0.0 |
| 12 | 0.00125 | +1.0 | 139 | 0.29 |
| 13 | 0.000625 | +1.5 | 291 | 0.62 |

## Patentansprüche

1. Pharmazeutisches Präparat zur Modulierung der Immunreaktion warmblütiger Tiere, dadurch gekennzeichnet, dass es als Wirkstoff ein Derivat der 2-Oxindol-3-essigsäure der Formel I, oder ein entsprechendes Derivat der 2-Oxo-1,2,3,4-tetrahydro-chinolin-4-carbonsäure der Formel II

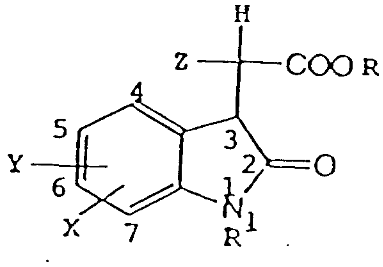

I

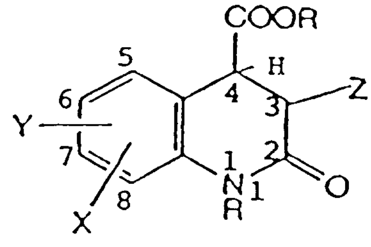

II

enthält, wobei die Verbindung der Formel I, gegebenenfalls mit der Verbindung der Formel II im Gleichgewicht steht und

$R^1$ ein Wasserstoffatom, einen Alkylrest mit 1-6 Kohlenstoffatomen, ein Aralkylrest mit 1-6 Kohlenstoffatomen im Alkylteil, einen Cycloalkylrest oder einen Acylrest der Formel,

$$-\overset{\overset{\displaystyle O}{\|}}{C}-R^4$$

darstellt, in welchem

$R^4$ ein Wasserstoffatom, ein Alkylrest mit 1-6 Kohlenstoffatomen, ein Aralkylrest mit 1-6 Kohlenstoffatomen im Alkylteil, ein Arylrest oder ein Cycloalkylrest ist,

Z ein Wasserstoffatom, ein Alkylrest mit 1-6 Kohlenstoffatomen, ein Aralkylrest mit 1-6 Kohlenstoffatomen in der Alkylgruppe, ein Cycloalkylrest oder ein Arylrest ist, und

X und Y unabhängig voneinander für Wasserstoffatome, Alkylreste, Cycloalkylreste, Arylreste, Hydroxygruppen, Halogenatome, Nitrogruppen, Ethergruppierungen der Formel

$-O-R^2$,

Estergruppierungen der Formel

$$-O-\overset{\overset{\displaystyle O}{\|}}{C}-R^4,$$

oder Estergruppierungen der Formel

$$-\underset{\underset{\displaystyle O}{\|}}{C}-O-R^2$$

stehen,

wobei in den oben angegebenen Formeln der Rest

$R^4$ die oben angegebenen Bedeutung besitzt und der Rest

$R^2$ ein Alkylrest mit 1-6 Kohlenstoffatomen, ein Aralkylrest mit 1-6 Kohlenstoffatomen im Alkylteil, ein Cycloalkylrest oder ein Arylrest ist oder die Reste X und Y gemeinsam eine Gruppierung der Formel

$-O-(CH_2)_n-O-$

darstellen, in welcher

n einen Wert von 1-4 aufweist und R ein Wasserstoffatom, ein Alkylrest mit 1-6 Kohlenstoffatomen, ein Aralkylrest mit 1-6 Kohlenstoffatomen im Alkylteil, ein Cycloalkylrest oder ein Arylrest ist oder der Rest R einen esterartig gebundenen Hexoserest oder eine Kette aus 2-6 Hexoseresten darstellt, wobei der Hexoserest, bzw. ein oder mehrere der Hexosereste, gegebenenfalls ausserdem Substituenten tragen, wie beispielsweise Hydroxylgruppen dieser Zuckerreste, die acyliert sind, und

wobei der Wirkstoff der Formel I, bzw. II, einschliesslich aller oben definierten Derivate in freier Form oder in Form von pharmazeutisch annehmbaren Salzen vorliegen, oder wobei der Wirkstoff eine Mischung darstellt, die mindestens zwei der oben definierten Derivate oder deren pharmazeutisch annehmbare Salze enthält.

**2.** Pharmazeutisches Präparat gemäss Anspruch 1, dadurch gekennzeichnet, dass es als Wirkstoff eine Verbindung der Formel I oder der Formel II enthält, in welcher

Z und $R^1$ ein Wasserstoffatom bedeuten,

R ein Wasserstoffatom, einen Alkylrest mit 1-6 Kohlenstoffatomen, einen Cycloalkylrest mit 3-8 Kohlenstoffatomen oder einen Arylrest, vorzugsweise einen Phenylrest oder einen substituierten Phenylrest bedeutet und

X und Y unabhängig voneinander für Wasserstoffatome, Hydroxygruppen, Halogenatome, Nitrogruppen oder Ethergruppierungen der Formel

$-O-R^2$

oder Estergruppierungen der Formel

$$-O-\overset{\displaystyle O}{\overset{\|}{C}}-R^4,$$

stehen, wobei in diesen Gruppierungen der Rest

$R^2$ einen Alkylrest mit 1-6 Kohlenstoffatomen oder einen Arylrest, vorzugsweise einen unsubstituierten oder substituierten Phenylrest darstellt und

$R^4$ ein Alkylrest mit 1-6 Kohlenstoffatomen, ein Arylrest, vorzugsweise ein unsubstituierter oder substituierter Phenylrest oder ein Cycloalkylrest mit 3-7 Kohlenstoffatomen ist.

**3.** Pharmazeutisches Präparat gemäss Anspruch 1 oder 2, dadurch gekennzeichnet, dass es als Wirkstoffe ein Racemat der Verbindung der Formel I oder ein Racemat der Verbindung der Formel II oder eine an einem der beiden optischen Antipoden angereicherte Form oder die Reinform des Antipoden der Verbindung der Formel I oder II enthält.

**4.** Pharmazeutisches Präparat gemäss Anspruch 2 oder 3, dadurch gekennzeichnet, dass es als Wirkstoff solche Verbindungen der Formel I, bzw. II enthält, in welchen sämtliche Reste R, Z, X, Y und $R^1$ die Bedeutung von Wasserstoffatomen besitzen.

**5.** Neue Verbindungen der Formel II, dadurch gekennzeichnet, dass sie die folgenden Formel IIb

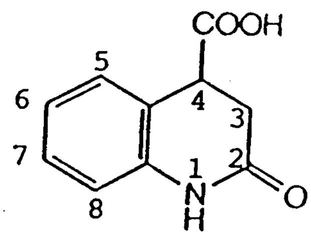

IIb

aufweisen, und dass sie in Form der optisch aktiven Enantiomeren vorliegen.

**6.** Neue chemische Verbindungen, dadurch gekennzeichnet, dass sie die Formel I oder II

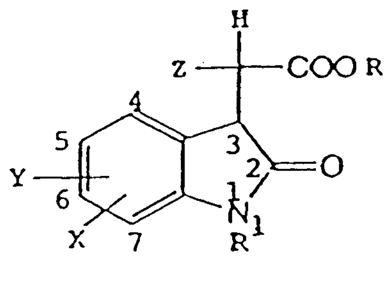

I

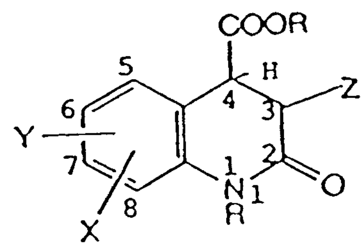

II

aufweisen,
wobei gegebenenfalls ein Gleichgewicht zwischen den Verbindungen der Formel I und den entsprechenden Verbindungen der Formel II vorliegt, wobei in diesen Verbindungen
$R^1$ ein Wasserstoffatom, einen Alkylrest mit 1-6 Kohlenstoffatomen, einen Aralkylrest mit 1-6 Kohlenstoffatomen im Alkylteil, einen Cycloalkylrest oder einen Acylrest der Formel

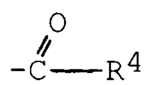

darstellt, in welchem
$R^4$ ein Wasserstoffatom, ein Alkylrest mit 1-6 Kohlenstoffatomen, ein Aralkylrest mit 1-6 Kohlenstoffatomen im Alkylteil, ein Arylrest oder ein Cycloalkylrest ist
Z ein Wasserstoffatom, ein Alkylrest mit 1-6 Kohlenstoffatomen, ein Aralkylrest mit 1-6 Kohlenstoffatomen in der Alkylgruppe, ein Cycloalkylrest oder ein Arylrest ist, und
X und Y unabhängig voneinander für Wasserstoffatome, Alkyreste, Cycloalkylreste, Arylreste, Hydroxygruppen, Halogenatome, Nitrogruppen, Ethergruppierungen der Formel

$-O-R^2$,

Estergruppierungen der Formel

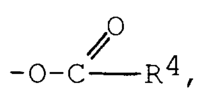

Estergruppierungen der Formel

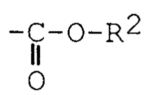

stehen, und wobei in den oben angegebenen Formeln der Rest
$R^4$ die oben angegebene Bedeutung besitzt und der Rest
$R^2$ ein Alkylrest mit 1-6 Kohlenstoffatomen, ein Aralkylrest mit 1-6 Kohlenstoffatomen im Alkylteil, ein Cycloalkylrest oder ein Arylrest ist oder die Reste X und Y gemeinsam eine Gruppierung der Formel

$-O-(CH_2)_n-O-$

darstellen, in welcher
n einen Wert von 1-4 aufweist und R ein Wasserstoffatom, ein Alkylrest mit 1-6 Kohlenstoffatomen,

ein Aralkylrest mit 1-6 Kohlenstoffatomen im Alkylteil, ein Cycloalkylrest oder ein Arylrest ist, oder einen esterartig gebundenen Hexoserest oder eine Kette aus 2-6 Hexoseresten darstellt, wobei der Hexoserest, bzw. ein oder mehrere der Hexosreste gegebenenfalls ausserdem Substituenten tragen, wie beispielsweise Hydroxylgruppen dieser Zuckerreste, die acyliert sind,

unter der Voraussetzung, dass in denjenigen Verbindungen der Formel I bzw. II, in welchen

R ein Wasserstoffatom oder ein Methylrest oder ein Ethylrest ist, und ferner

$R^1$ Wasserstoff oder Methyl bedeutet und ausserdem

Z Wasserstoff ist,

die Reste X und Y nicht gemeinsam zwei Wasserstoffatome, zwei Chloratome, zwei Bromatome, zwei Methoxygruppen oder die Gruppe der Formel $-O-CH_2-O-$ bedeuten dürfen, und ausserdem dann, wenn der Rest X Wasserstoff ist, der Rest Y eine andere Bedeutung aufweisen muss als diejenige eines Jodatoms, einer Hydroxygruppe, einer Methoxygruppe oder einer Methylgruppe und

unter der weiteren Voraussetzung, dass in denjenigen Verbindungen der Formel I oder II, in welchen Z, X und Y ein Wasserstoffatom bedeuten, R Wasserstoff, Methyl, Ethyl oder Butyl ist, der Rest

$R^1$ eine andere Bedeutung aufweisen muss als diejenige eines Methylrestes, eines Butylrestes, eines Benzylrestes, eines Acetylrestes oder eines 4-Chlorbenzoylrestes und

unter der weiteren Voraussetzung, dass in denjenigen 2-Oxo-1,2,3,4-tetrahydrochinolin-4-carbonsäuren der Formel II, in welchen Z ein Ethylrest ist, mindestens einer der Reste R, $R^1$, X und Y eine andere Bedeutung aufweisen muss als diejenige eines Wasserstoffatomes,

sowie Salze dieser neuen Wirkstoffe der Formeln I bzw. II.

**7.** Neue chemische Verbindungen gemäss Anspruch 6, dadurch gekennzeichnet, dass sie Ester der Formel I, bzw. II sind, wobei in diesen

R ein Alkylrest mit 3-6 Kohlenstoffatomen, ein Aralkylrest mit 1-6 Kohlenstoffatomen im Alkylteil, in welchem der Arylrest ein Phenylrest oder ein substituierter Phenylrest ist, ein Cycloalkylrest mit 3-7 Kohlenstoffatommen, ein Arylrest, vorzugsweise ein substituierter oder unsubstituierter Phenylrest ist, oder R einen esterartig gebundenen Hexoserest oder eine Gruppe aus 2-6 Hexoseresten, beispielsweise Glucosereste, wobei diese Hexosereste gegebenenfalls noch substituiert sind, darstellt, und wobei

die Reste $R^1$, Z,X und Y die in Anspruch 6 angegebene Bedeutung besitzen, wobei jedoch

dann, wenn $R^1$ ein Butylrest ist und Z, X und Y für Wasserstoff stehen, der Rest R eine andere Bedeutung aufweisen muss als diejenige eines Butylrestes,

und wobei diese Verbindungen der Formel I oder II in freier Form oder in Form von Salzen vorliegen.

**8.** Verbindungen der Formel I, bzw. II, gemäss Anspruch 6, dadurch gekennzeichnet, dass in ihnen

R Wasserstoff bedeutet,

Z ein Alkylrest mit 1-6 Kohlenstoffatomen, ein Aralkylrest mit 1-6 Kohlenstoffatomen in der Alkylgruppe, in welchem der Arylrest vorzugsweise ein unsubstituierter oder substituierter Phenylrest ist, ein Cycloalkylrest mit 3-7 Kohlenstoffatomen oder ein Arylrest, vorzugsweise ein substituierter oder unsubstituierter Phenylrest ist und

X, Y und $R^1$ die im Anspruch 6 angegebenen Bedeutungen besitzen,

unter der Voraussetzung, dass dann, wenn in diesen Säuren Z ein Ethylrest ist, mindestens einer der Substituenten $R^1$, X und Y eine andere Bedeutung aufweisen muss als diejenige eines Wasserstoffatomes und

wobei diese Verbindungen der Formel I, bzw. II in freier Form oder in Form von Salzen vorliegen.

**9.** Verbindungen der Formel I, bzw. II, gemäss Anspruch 6, dadurch gekennzeichnet, dass

die Reste R, Z und $R^1$ die in Anspruch 6 angegebenen Bedeutungen besitzen und mindestens einer der Reste

X und Y die Bedeutung von Alkylresten mit mindestens 2 Kohlenstoffatomen, Cycloalkylresten, Phenylresten, Nitrogruppen, Alkoxyreste mit mindestens 2 Kohlenstoffatomen, oder Estergruppierungen der Formel

$$-O-\overset{\overset{\displaystyle O}{\|}}{C}-R^4$$

darstellt, wobei in dieser Gruppierung

$R^4$ ein Alkylrest mit 1-6 Kohlenstoffatomen, ein Arylrest, vorzugsweise ein unsubstituierter oder substituierter Phenylrest oder ein Cycloalkylrest mit 3-7 Kohlenstoffatomen ist,

wobei jedoch dann, wenn mindestens einer der Reste R, Z oder $R^1$ eine andere Bedeutung aufweist als diejenige eines Wasserstoffatoms, der Rest X und/oder der Rest Y ausserdem die Bedeutung von Methylresten und/oder Methoxyresten aufweisen kann, wobei jedoch dann, wenn in den Verbindungen der Formel I, Z und $R^1$ Wasserstoff bedeuten und R Methyl ist und beide Reste X und Y für Methoxygruppen stehen, sich diese nicht in den Stellungen 5 und 6 des Benzolkernes des Indolgerüstes der Verbindungen der Formel I befinden dürfen.

Europäisches Patentamt

# EUROPÄISCHER RECHERCHENBERICHT

Nummer der Anmeldung
EP 95 10 7887

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| X | CHEMICAL ABSTRACTS, vol. 103, no. 23, 9.Dezember 1985<br>Columbus, Ohio, US;<br>abstract no. 193222a,<br>NONHEBEL, HEATHER M. ET AL. 'Indole-3-acetic acid ...'<br>* Zusammenfassung *<br>* RN 99102-27-9 *<br>& J. BIOL. CHEM. ,<br>Bd. 260, Nr. 23, 1985<br>Seiten 12685-12689,<br>----- | 6,7 | C07D209/34<br>C07D215/50<br>A61K31/40<br>A61K31/47 |
| | | | RECHERCHIERTE SACHGEBIETE (Int.Cl.6) |
| | | | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 26.Juli 1995 | Van Bijlen, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)